# EUROPEAN PATENT SPECIFICATION

(11) **EP 2 536 466 B1**
(45) Date of publication and mention of the grant of the patent: **26.12.2018**
(21) Application number: 11704520.3
(22) Date of filing: 15.02.2011
(51) Int. Cl.: A61N 1/365, A61N 1/362, A61B 5/0464, A61B 5/0205, A61B 5/08, A61N 1/39

(54) **HEMODYNAMIC STABILITY DETECTION DURING ARRHYTHMIA USING RESPIRATION SENSOR**
NACHWEIS VON HÄMODYNAMISCHER STABILITÄT BEI ARRHYTHMIEN MIT EINEM ATEMSENSOR
DÉTECTION DE LA STABILITÉ HÉMODYNAMIQUE PENDANT UNE ARYTHMIE EN UTILISANT UN CAPTEUR DE RESPIRATION

(30) Priority: 17.02.2010 US 305258 P
(43) Date of publication of application: 26.12.2012
(73) Proprietor: Cardiac Pacemakers, Inc., St. Paul, MN 55112-5798 (US)
(72) Inventor: LI, Dan, Shoreview, MN 55126 (US); SHARMA, Arjun, St. Paul, MN 55102 (US); ZHANG, Yi, Plymouth, MN 55446 (US)
(74) Representative: Pfenning, Meinig & Partner mbB
(86) International application number: PCT/US2011/024845
(87) International publication number: WO 2011/103071

(56) References cited:
- US-A- 5 113 869
- US-A1- 2004 225 332
- US-A1- 2006 259 089
- US-A1- 2007 149 890
- US-A1- 2007 239 218
- US-A1- 2007 293 781
- US-A1- 2009 131 999

## Description

### BACKGROUND

Implantable medical devices (IMDs) are devices designed to be implanted into a patient. Some examples of these devices include cardiac rhythm management (CRM) devices. CRM devices include implantable pacemakers, implantable cardioverter defibrillators (ICDs), and devices that include a combination of pacing and defibrillation including cardiac resynchronization therapy. The devices are typically used to treat patients using electrical therapy and to aid a physician or caregiver in patient diagnosis through internal monitoring of a patient's condition. The devices can include electrical leads in communication with sense amplifiers to monitor electrical heart activity within a patient, and often include sensors to monitor other internal patient parameters. Other examples of implantable medical devices include implantable insulin pumps or devices implanted to administer drugs to a patient.

Additionally, some IMDs detect events by monitoring electrical heart activity signals. By monitoring cardiac signals, IMDs are able to detect abnormally rapid heart rate, or tachyarrhythmia. Although detecting an occurrence of tachyarrhythmia is important, it can be even more helpful if additional physiologic information is known about the tachyarrhythmia, such as if the tachyarrhythmia is hemodynamically stable or unstable. Hemodynamically stable tachyarrhythmia does not cause a significant drop in the patient's blood pressure or cardiac output, and it can generally be treated with anti-tachyarrhythmia pacing (ATP).
or regional perfusion is not adequate to support normal organ function. Hemodynamically unstable tachyarrhythmia generally requires shock therapy or cardioversion. Thus, an IMD that can not only detect tachyarrhythmias, but also discriminate between hemodynamically stable and unstable tachyarrhythmias, can be used to help guide therapy decisions.

US 2007/0149890 discloses a tachyarrhythmia detection and classification system classifying tachyarrhythmias based on an analysis of morphological features of a cardiac signal enhanced by using one or more physiological parameters indicative of hemodynamic stability and/or activity level. The tachyarrhythmia detection and classification system computes a measure of similarity between an arrhythmic waveform of the cardiac signal and a template waveform for that cardiac signal such as a correlation coefficient representative of the correlation between morphological features of the arrhythmic waveform and morphological features of the template waveform.

US 2009/0131999 discloses a system for sensing, during an event of tachycardia, hemodynamic signals concurrently from at least two spatially separated locations within a patient and quantifying a spatial relationship between the hemodynamic signals. Based on the analysis one or more anti-tachycardia therapies may be elicited. The hemodynamic signals used may be heart sound signals or accelerometers, microphones or pressure sensors.

### OVERVIEW

The present invention provides an apparatus for determining a hemodynamic stability characteristic of an arrhythmia as defined in claim 1. Preferred embodiments are defined by the dependent claims.

Aspects, embodiments or examples of the present disclosure which do not fall within the scope of the appended claims do not form part of the present invention and are presented for illustrative purposes only.

This document describes, among other things, systems and methods for discriminating between hemodynamically stable and hemodynamically unstable tachyarrhythmias using detected changes in respiration parameters, either alone or in conjunction with other physiological signals.

Example I can include subject matter that can include an apparatus comprising: a cardiac rhythm management device comprising: a tachyarrhythmia detection circuit configured to detect whether tachyarrhythmia is present in a subject; a respiration sensing circuit, coupled to the tachyarrhythmia detection circuit, configured to sense, during the tachyarrhythmia, a respiration signal from the subject; an auxiliary sensing circuit, coupled to the tachyarrhythmia detection circuit, configured to sense, during the tachyarrhythmia, an auxiliary physiological signal from the subject; and a processor circuit, coupled to the respiration sensing circuit and the auxiliary sensing circuit, the processor circuit configured to: determine a characteristic of the respiration signal; determine a characteristic of the auxiliary physiological signal; determine a measure of concordance between the characteristic of the respiration signal and the characteristic of the auxiliary physiological signal; and use the measure of concordance to determine a hemodynamic stability characteristic of the tachyarrhythmia.

In Example 2, the subject matter of Example 1 can optionally include the processor circuit configured to determine the characteristic of the respiration and auxiliary physiological signals, respectively, determined over multiple cardiac cycles.

In Example 3, the subject matter of any one of Examples 1-2 can optionally include the auxiliary sensing circuit configured to sense at least one of a physical activity level, a heart rate, a heart sound, or an acceleration.

In Example 4, the subject matter of any one of Examples 1-3 can optionally include the processor configured to determine the characteristic of the respiration signal by determining at least one of: a time rate of change of a respiration rate, a time rate of change of a respiration depth, or a time rate of change of a respiration morphological pattern; wherein the processor is configured to determine the characteristic of the respiration signal using the characteristic of the respiration signal determined over multiple cardiac cycles.

In Example 5, the subject matter of any one of Examples 1-4 can optionally include the processor configured to: compare the characteristic of the respiration signal to a first threshold value; compare the characteristic of the auxiliary physiological signal to a second threshold value; and use the comparisons to determine an indication of at least one of concordance or discordance.

In Example 6, the subject matter of any one of Examples 1-5 can optionally include the processor configured to determine discordance when the characteristic of the respiration signal exceeds the first threshold value and the characteristic of the auxiliary physiological signal is less than the second threshold value.

In Example 7, the subject matter of any one of Examples 1-6 can optionally include the processor configured to declare that the tachyarrhythmia is hemodynamically unstable in response to a determined discordance.

In Example 8, the subject matter of any one of Examples 1-7 can optionally include the processor configured to determine concordance when: 1) the characteristic of the respiration signal exceeds the first threshold value and the characteristic of the auxiliary physiological signal exceeds the second threshold value; or 2) the characteristic of the respiration signal is less than the first threshold value and the characteristic of the auxiliary physiological signal is less than the second threshold value.

In Example 9, the subject matter of any one of Examples 1-8 can optionally include the processor configured to declare that the tachyarrhythmia is hemodynamically stable in response to a determined concordance.

In Example 10, the subject matter of any one of Examples 1-9 can optionally include the hemodynamic stability characteristic of the tachyarrhythmia including one of hemodynamic stability or hemodynamic instability.

In Example 11, the subject matter of any one of Examples 1-10 can optionally include the processor configured to communicate an indication of the hemodynamic stability characteristic of the tachyarrhythmia to a user interface or process.

In Example 12, the subject matter of any one of Examples 1-11 can optionally include the processor configured to use the hemodynamic stability characteristic of the tachyarrhythmia to control therapy provided to the subject.

In Example 13, the subject matter of any one of Examples 1-12 can optionally include a therapy circuit, coupled to the processor circuit, the therapy circuit configured to provide anti-tachyarrhythmia pacing to the subject when the tachyarrhythmia is hemodynamically stable.

In Example 14, the subject matter of any one of Examples 1-13 can optionally include a therapy circuit, coupled to the processor circuit, the therapy circuit configured to provide shock therapy to the subject when the tachyarrhythmia is hemodynamically unstable.

In Example 15, the subject matter of any one of Examples 1-14 can optionally include a therapy circuit, coupled to the processor circuit, the therapy circuit configured to withhold shock therapy when the tachyarrhythmia is hemodynamically stable.

Example 16 can include, or can optionally be combined with any one of Examples 1-15 to include subject matter that can include determining that a tachyarrhythmia is present in a subject; sensing a respiration signal from the subject during the tachyarrhythmia; sensing an auxiliary physiological signal, other than the respiration signal, from the subject during the tachyarrhythmia; determining a characteristic of the respiration signal; determining a characteristic of the auxiliary physiological signal; determining a measure of concordance between the characteristic of the respiration signal and the characteristic of the auxiliary physiological signal; and using the measure of concordance to determine a hemodynamic stability characteristic of the tachyarrhythmia.

In Example 17, the subject matter of any one of Examples 1-16 can optionally include determining the characteristic of the respiration and auxiliary physiological signals, respectively, determined over multiple cardiac cycles.

In Example 18, the subject matter of any one of Examples 1-17 can optionally include sensing at least one of a physical activity level, a heart rate, a heart sound, or an acceleration.

In Example 19, the subject matter of any one of Examples 1-18 can optionally include determining at least one of: a time rate of change of a respiration rate, a time rate of change of a respiration depth, or a time rate of change of a respiration morphological pattern; and wherein determining the characteristic of the respiration signal includes determining the characteristic of the respiration signal determined over multiple cardiac cycles.

In Example 20, the subject matter of any one of Examples 1-19 can optionally include comparing the characteristic of the respiration signal to a first threshold value; comparing the characteristic of the auxiliary physiological signal to a second threshold value; and using the comparisons to determine an indication of at least one of concordance or discordance.

In Example 21, the subject matter of any one of Examples 1-20 can optionally include determining discordance when the characteristic of the respiration signal exceeds the first threshold value and the characteristic of the auxiliary physiological signal is less than the second threshold value.

In Example 22, the subject matter of any one of Examples 1-21 can optionally include declaring that the tachyarrhythmia is hemodynamically unstable in response to a determined discordance.

In Example 23, the subject matter of any one of Examples 1-22 can optionally include determining concordance when: 1) the characteristic of the respiration signal exceeds the first threshold value and the characteristic of the auxiliary physiological signal exceeds the second threshold value; or 2) the characteristic of the respiration signal is less than the first threshold value and the characteristic of the auxiliary physiological signal is less than the second threshold value.

In Example 24, the subject matter of any one of Examples 1-23 can optionally include declaring that the tachyarrhythmia is hemodynamically stable in response to a determined concordance.

In Example 25, the subject matter of any one of Examples 1-24 can optionally include the hemodynamic stability characteristic of the tachyarrhythmia including one of hemodynamic stability or hemodynamic instability.

In Example 26, the subject matter of any one of Examples 1-25 can optionally include communicating an indication of the hemodynamic stability characteristic of the tachyarrhythmia to a user interface or process.

In Example 27, the subject matter of any one of Examples 1-26 can optionally include using the hemodynamic stability characteristic of the tachyarrhythmia to control therapy provided to the subject.

In Example 28, the subject matter of any one of Examples 1-27 can optionally include providing anti-tachyarrhythmia pacing to the subject when the tachyarrhythmia is hemodynamically stable.

In Example 29, the subject matter of any one of Examples 1-28 can optionally include providing shock therapy to the subject when the tachyarrhythmia is hemodynamically unstable.

In Example 30, the subject matter of any one of Examples 1-29 can optionally include withholding shock therapy when the tachyarrhythmia is hemodynamically stable.

Example 31 can include, or can optionally be combined with any one of Examples 1-30 to include subject matter that can include an apparatus comprising: a cardiac rhythm management device comprising: a tachyarrhythmia detection circuit configured to detect whether tachyarrhythmia is present in a subject; a respiration sensing circuit, coupled to the tachyarrhythmia detection circuit, configured to sense a respiration signal from the subject; and a processor circuit, coupled to the respiration sensing circuit, the processor circuit configured to: determine a characteristic of a respiration rate or interval from the respiration signal; compare the characteristic of the respiration rate or interval to a specified threshold value; when the characteristic of the respiration rate exceeds the specified threshold value, declare that the tachyarrhythmia is hemodynamically unstable.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. **1** is a schematic diagram illustrating generally an example of an implantable or other ambulatory cardiac rhythm management (CRM) device.
FIG. **2** is a block diagram illustrating generally an example of portions of the CRM device electronics unit.
FIGS. **3A** and **3B** illustrate examples of conceptualized (not real) data that can be used to determine a relationship, such as a measure of concordance, between respiratory rate and an auxiliary physiological signal.
FIG. **4** illustrates an example of a method for differentiating between stable and unstable tachyarrhythmia.
FIG. **5** illustrates an example of another method for differentiating between stable and unstable tachyarrhythmia.

### DETAILED DESCRIPTION

The present inventors have recognized, among other things, that changes in respiration parameters, in conjunction with other physiological signals, can be used to discriminate between hemodynamically stable and hemodynamically unstable tachyarrhythmias. Respiration signals can be detected by minute ventilation sensors or other respiration sensors. Discrimination of hemodynamically stable versus unstable tachyarrhythmias can help guide therapy decisions.

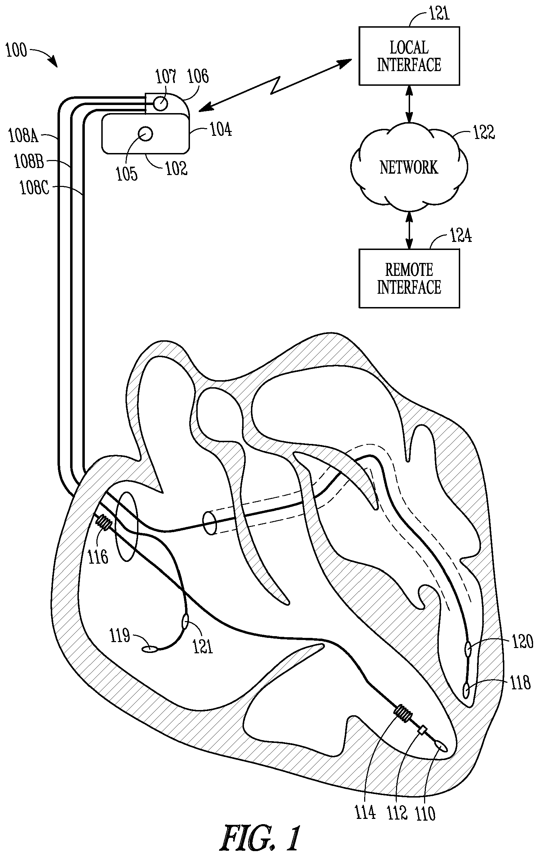

FIG. 1 shows an example of an implantable or other ambulatory cardiac rhythm management (CRM) device **100.** In an example, the CRM device **100** can include an electronics unit **102** that can include a hermetically-sealed biocompatible housing **104** and a header **106** extending therefrom. The housing **104** can carry a power source and electronics. The header **106** can include one or more receptacles, such as for receiving the proximal ends of intravascular leads **108A-C.** In an example, the lead **108A** can be an intravascular RV lead that can extend from the superior vena cava (SVC) into the right atrium (RA), and then into the right ventricle (RV). The lead **108A** can include an RV apical tip electrode **110,** a slightly more proximal RV ring electrode **112,** a still slightly more proximal RV shock coil electrode **114,** and an even more proximal RA or SVC shock coil electrode **116.** The various electrodes can be used for delivering electrical energy or sensing intrinsic electrical heart signals. An intravascular CS/LV lead **108C** can extend from the SVC into the RA, through a coronary sinus (CS) into the coronary vasculature, such as near a portion of a left ventricle (LV). In an example, this second CS/LV lead **108B** can include at least a distal electrode **118** and a proximal electrode **120,** from which electrostimulation energies can be delivered or intrinsic electrical heart signals can be sensed. An intravascular right atrial (RA) lead **108B** can extend from the SVC into the RA, and can include a distal electrode **119** and a proximal electrode **121.** Other electrodes (e.g., a housing electrode **105** on the housing **104,** a header electrode **107** on the header **106,** an epicardial electrode, a subcutaneous electrode located away from the heart, or an electrode located elsewhere) or leads can be used.

In an example, an implantable CRM device **100** can include a communication circuit, such as to wireless communicate unidirectionally or bidirectionally with an external local interface **121,** such as a CRM device programmer, repeater, handheld device, or the like. The local interface **121** can be configured to communicate via a wired or wireless computer or communication network **122** to a remote interface **124,** such as a remote computer or server or the like.

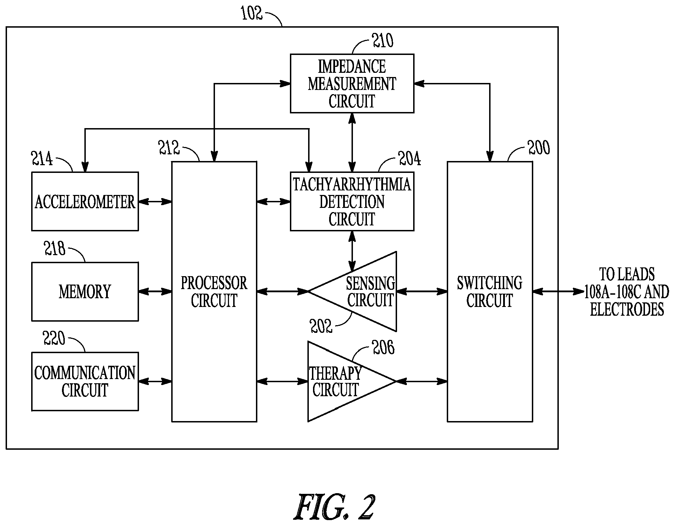

FIG. **2** shows an example of portions of the CRM device electronics unit **102.** In an example, this can include a switching circuit **200,** such as for selectively connecting to the various electrodes such as on the leads **108A-C** or elsewhere. A sensing circuit **202** can be selectively coupled to various electrodes by the switching circuit **200,** and can include sense amplifiers, filter circuits, other circuits such as for sensing intrinsic electrical signals, such as intrinsic heart signals. The sensing circuit **202** can be coupled to a tachyarrhythmia detection circuit **204.** The tachyarrhythmia detection circuit **204** can be configured to detect tachyarrhythmia in a patient, such as by using heart rate or morphology information from the depolarizations sensed by the sensing circuit **202.** A therapy circuit **206** can be selectively coupled to various electrodes by the switching circuit **200,** and can include pacing energy generation circuitry (e.g., capacitive, inductive, or other) such as for generating, storing, or delivering an electrostimulation, cardioversion, defibrillation, or other energy. An impedance measurement circuit **210** can be selectively coupled to various electrodes by the switching circuit **200,** such as for measuring a lead impedance, a tissue impedance, a regional or organ impedance, or other impedance. Impedance measurements, such as transthoracic impedance measurements, can be used, for example, to detect a respiration signal, such as respiration rate, respiration depth, or a respiration morphological pattern. Impedance measurements can also be used to detect a fluid status. The impedance measurement circuit **210** can be coupled to the tachyarrhythmia detection circuit **204.** In an example, the sensing circuit **202,** the tachyarrhythmia detection circuit **204,** the therapy circuit **206,** or the impedance measurement circuit **210** can be coupled to a processor circuit **212.** In an example, the processor **212** can perform instructions, such as for signal processing of signals derived by the sensing circuit **202,** the tachyarrhythmia detection circuit **204,** or the impedance circuit **210,** or for controlling operation of the therapy circuit **206** or other operations of the CRM device **100.** The processor **212** can be coupled to or include a physical activity sensor, such as an accelerometer **214,** configured to sense a patient's acceleration or physical activity level. The accelerometer **214** can be coupled to the tachyarrhythmia detection circuit **204.** In an example, the accelerometer **214** can be configured to sense other measures of physical activity, such as heart sounds. In an example, the accelerometer **214** can be configured to detect respiration signals by detecting muscle stretch or movement of the diaphragm or intercostal muscles. Although not shown in FIG. **2****,** other sensors, in addition to the accelerometer **214** and the impedance measurement circuit **210,** can be configured to detect respiration signals. Examples of such other sensors configured to detect respiration signals include acoustic or ultrasound sensors, as well as blood pressure or blood flow sensors. The processor **212** can also be coupled to or include a memory circuit **218,** such as for storing or retrieving instructions or data, or a communication circuit **220,** such as for communicating with the local interface **121.**

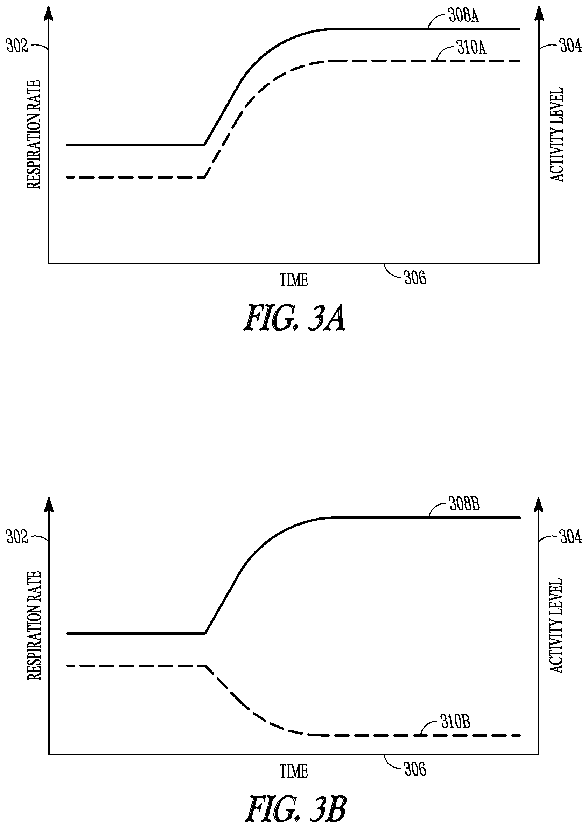

FIGS. **3A** and **3B** illustrate examples of conceptualized (not real) data that can be used to determine a relationship, such as a measure of concordance, between respiratory signal, such as respiratory rate, and an auxiliary physiological signal, such as physical activity level. Other respiratory signals that can be used, instead of or in addition to respiratory rate, include respiration depth or respiration morphological pattern. Other auxiliary physiological signals that can be used, instead of or in addition to physical activity level, include heart rate, heart sounds, or acceleration, for example. In the examples illustrated, the relationships between respiratory rate and physical activity level can be used to differentiate hemodynamically stable tachyarrhythmia from hemodynamically unstable tachyarrhythmia. As described with respect to FIG. **2** above, a respiration signal can be detected, for example, by the impedance measurement circuit **210** or the accelerometer **214,** and the physical activity level can be detected by the accelerometer **214.** In an example, by comparing the time rate of change of the respiratory rate to the time rate of change of the physical activity level, a measure of concordance can be determined. In FIGS. **3A** and **3B****,** y-axis **302** represents respiratory rate and y-axis **304** represents physical activity level. Both respiratory rate and physical activity level are plotted against time, which is represented by the x-axis **306.** In FIG. **3A****,** respiratory rate, represented by line **308A,** increases concordantly with activity level, represented by line **310A.** Concordance of respiratory rate and activity level can be observed under normal physiological conditions, such as during exercise. Concordance of respiratory rate and activity level during a tachyarrhythmia can be indicative of a hemodynamically stable tachyarrhythmia. In Fig. **3B****,** respiratory rate, represented by line **308B,** increases while activity level, represented by line **310B,** decreases. The resulting discordance of respiratory rate and activity level can be indicative of a hemodynamically unstable tachyarrhythmia, which, in turn, can result in hemodynamic deterioration.

In an example, the relationship, indicative of a hemodynamic stability characteristic, between a characteristic of a respiration signal and a characteristic of a physical activity level, can be defined as an interdependence between the characteristic of the respiration signal and the characteristic of the physical activity level. Thus, for example, in addition to or instead of a measure of concordance, a relationship can be determined between respiratory rate and physical activity level by comparing a respiration rate corresponding to a specified activity level to a threshold value. In an example, a respiration rate that is above a threshold value for a specified activity level can be indicative of a hemodynamically unstable tachyarrhythmia. Likewise, a respiration rate that is below a threshold value for a specified activity level can be indicative of a hemodynamically stable tachyarrhythmia.

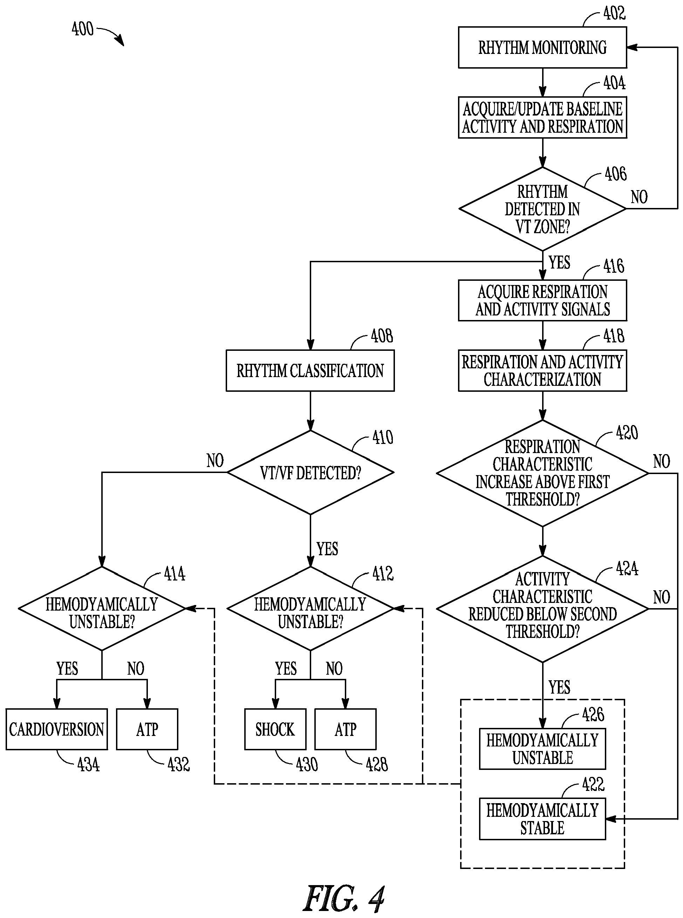

FIG. **4** illustrates an example of a method **400** for differentiating between stable and unstable tachyarrhythmia. At **402,** cardiac rhythm is detected and monitored, such as by an implantable CRM device. At **404,** baseline respiration and physical activity level measurements are acquired or updated. In an example, other auxiliary physiological signals, such as heart rate, heart sounds, or posture, can be monitored in addition to or instead of physical activity. At **406,** if the detected cardiac rhythm is in the ventricular tachyarrhythmia zone, such as 120 to 200 beats per minute (bpm), then at **408,** the detected tachyarrhythmia is classified. The tachyarrhythmia can be classified, for example, via analysis of the detected electrophysiological signal. At **410,** if the tachyarrhythmia is classified as either ventricular tachyarrhythmia or ventricular fibrillation, then the process flows to **412.** In an example, when the tachyarrhythmia is classified as ventricular fibrillation (e.g., >200 bpm), the hemodynamic stability determination at **412** can be skipped, and the process can flow directly to **430,** where shock therapy is delivered to the patient. At **410,** if the tachyarrhythmia is not classified as either ventricular tachyarrhythmia or ventricular fibrillation, then the process flows to **414.**

At **406,** if the detected cardiac rhythm is in the ventricular tachyarrhythmia zone, then at **416** respiration and physical activity signals are acquired during the tachyarrhythmia. Respiration and activity signals can be acquired at **416** concurrently with classification of the tachyarrhythmia at **408.** In an example, respiration and activity signals can be acquired at **416** continuously. In an example, respiration and activity signals can be acquired at **416** only if specified respiration sensor activation conditions are met. In an example, a condition for respiration sensor activation can be a detected heart rate that is below the lower limit of the ventricular tachyarrhythmia zone but above a specified threshold value. This can allow for the detection and treatment of slow ventricular tachyarrhythmias. Another example of a condition for respiration sensor activation can include a cardiac cycle length stability that is below a specified threshold value, such as 20 milliseconds. Still other examples of conditions for respiration sensor activation include the presence of specified electrocardiogram characteristics, the status of therapy delivery (e.g. anti-tachyarrhythmia pacing), a detected drop in intracardiac impedance that is below a specified threshold value, or manual activation by a user or healthcare provider.

At **418,** the respiration and activity signals are characterized. In an example, characterization of the respiration and activity signals can occur on an ongoing basis. In an example, characterization of the respiration and activity signals can occur only if specified respiration sensor activation conditions are met, such as those described above. The respiration signal can be characterized, for example, by determining a time rate of change of a respiration rate, a time rate of change of a respiration depth, or a time rate of change of a respiration morphological pattern. In an example, the respiration signal can be characterized by determining a change in correlation or a change in coherence among multiple respiration signal measurements within a specified number of heart beats. The physical activity signal can be characterized, for example, by determining a physical activity level, a heart rate, a heart sound, or an acceleration. In an example, the respiration and physical activity signals can be characterized over multiple cardiac cycles. At **420,** if the respiration characteristic is not increased above a first threshold value, then at **422** the tachyarrhythmia is declared hemodynamically stable. At **420,** if the respiration characteristic is increased above the first threshold value, then the process flows to **424.** In an example, the respiration characteristic can be measured and compared to the first threshold value after a specified time period, such as 5-10 seconds from the onset of the tachyarrhythmia. At **424,** if the activity characteristic is not reduced below a second threshold, then at **422** the tachyarrhythmia is declared hemodynamically stable. At **424,** if the activity characteristic is reduced below the second threshold, then at **426,** the tachyarrhythmia is declared hemodynamically unstable. In an example, the activity characteristic can be measured and compared to the second threshold value after a specified time period, such as 5-10 seconds from the onset of the tachyarrhythmia. After the tachyarrhythmia has been declared hemodynamically stable at **422** or unstable at **426,** the process flows back to **412** and **414.** At **412,** if the tachyarrhythmia has been declared hemodynamically stable, then at **428** anti-tachyarrhythmia pacing (ATP) is provided to the patient. At **412,** if the tachyarrhythmia has been declared hemodynamically unstable, then at **430** shock therapy is provided to the patient. At **414,** if the tachyarrhythmia has been declared hemodynamically stable, then at **432** ATP is provided to the patient. At **414,** if the tachyarrhythmia has been declared hemodynamically unstable, then at **434** cardioversion is provided to the patient.

In an example, at **416,** a respiration signal can be acquired without acquiring an activity signal. At **418,** the respiration signal can be characterized, such as by determining a respiration rate or interval, a tidal volume, a depth of respiration, or a respiratory pattern. In an example, the respiration signal can be characterized by determining a time rate of change or a relative change of a respiration rate, a tidal volume, a respiration depth, or a respiratory pattern. At **420,** the respiration characteristic can be compared to a specified threshold value. If the respiration characteristic exceeds the specified threshold value, then at **426,** the tachyarrhythmia is declared hemodynamically unstable. If the respiration characteristic does not exceed the threshold value, then at **422** the tachyarrhythmia is declared hemodynamically stable. In an example, at **420,** the respiration characteristic can be compared to a specified criteria instead of, or in addition to, being compared to a specified threshold value. If the respiration characteristic meets the specified criteria, then at **426,** the tachyarrhythmia is declared hemodynamically unstable. If the respiration characteristic does not meet the specified criteria, then at **422** the tachyarrhythmia is declared hemodynamically stable. After the tachyarrhythmia has been declared hemodynamically stable at **422** or unstable at **426,** the process flows back to **412** and **414,** as described above.

An example of a situation in which the respiration characteristic alone (e.g., without a physical activity characteristic) can be used to distinguish hemodynamically stable and unstable tachyarrhythmias includes when the patient's detected heart rate is above a specified threshold value (e.g. 180 bpm). In this situation, it is unlikely that physical activity would result in or contribute to such a high heart rate. Therefore, it would be unnecessary to detect the physical activity characteristic in conjunction with the respiration characteristic in order to determine hemodynamic stability or instability; rather, analysis of the respiration characteristic alone would suffice to differentiate hemodynamically stable and unstable tachyarrhythmias. For example, a respiration rate that is increased above a specified threshold can be indicative of hemodynamically unstable tachyarrhythmia, and a respiration rate that is not increased above a specified threshold can be indicative of hemodynamically stable tachyarrhythmia.

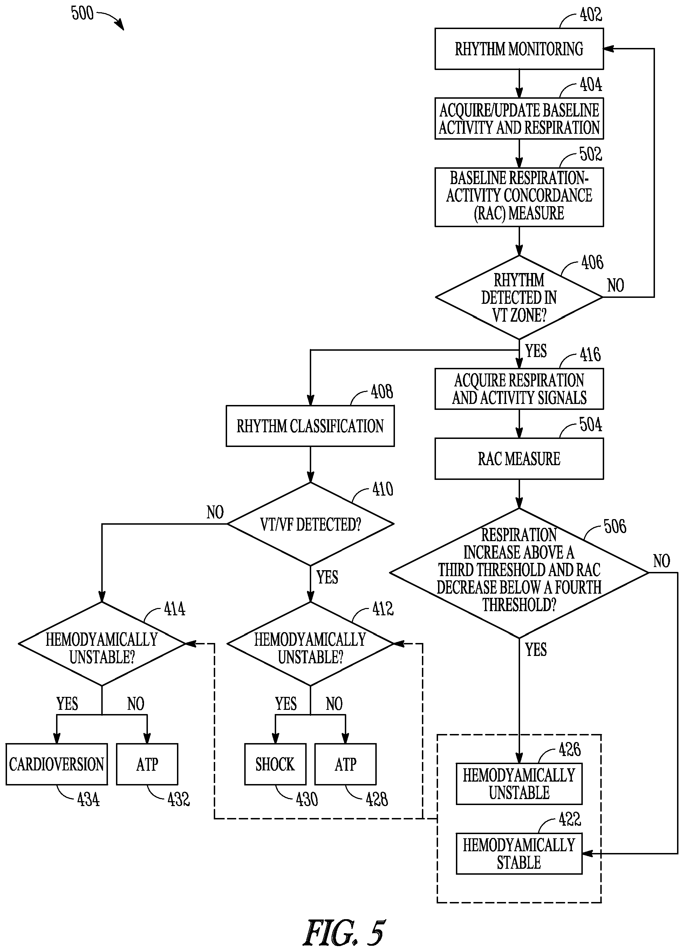

FIG. **5** illustrates an example of another method **500** for differentiating between stable and unstable tachyarrhythmia. At **402,** cardiac rhythm is detected and monitored, such as by an implantable CRM device. At **404,** baseline respiration and physical activity level measurements are acquired or updated. In an example, other auxiliary physiological signals, such as heart rate, heart sounds, or posture, can be monitored in addition to or instead of physical activity. At **502,** a baseline Respiration-Activity Concordance (RAC) measure is obtained. The RAC measure can be obtained, for example, using plotted data such as that shown in FIGS. **3A** and **3B****.** In an example, the RAC measure can be a ratio of the respiratory rate measured at a specified time to the activity level measured at the same specified time. In an example, the RAC measure can be a ratio can be a ratio of the rate of change of the respiratory rate to the rate of change of the activity level. At **406,** if the detected cardiac rhythm is in the ventricular tachyarrhythmia zone, such as 120 to 200 bpm, then at **408,** the detected tachyarrhythmia is classified. The tachyarrhythmia can be classified, for example, via analysis of the detected electrophysiological signal. At **410,** if the tachyarrhythmia is classified as either ventricular tachyarrhythmia or ventricular fibrillation, then the process flows to **412.** In an example, when the tachyarrhythmia is classified as ventricular fibrillation (e.g., >200 bpm), the hemodynamic stability determination at **412** can be skipped, and the process can flow directly to **430,** where shock therapy is delivered to the patient. At **410,** if the tachyarrhythmia is not classified as either ventricular tachyarrhythmia or ventricular fibrillation, then the process flows to **414.**

At **406,** if the detected cardiac rhythm is in the ventricular tachyarrhythmia zone, then at **416** respiration and physical activity signals are acquired during the tachyarrhythmia. Respiration and activity signals can be acquired at **416** concurrently with classification of the tachyarrhythmia at **408.** In an example, respiration and activity signals can be acquired at **416** continuously. In an example, respiration and activity signals can be acquired at **416** only if specified respiration sensor activation conditions are met. In an example, a condition for respiration sensor activation can be a detected heart rate that is below the lower limit of the ventricular tachyarrhythmia zone but above a specified threshold value. This can allow for the detection and treatment of slow ventricular tachyarrhythmias. Another example of a condition for respiration sensor activation can include a cardiac cycle length stability that is below a specified threshold value, such as 20 milliseconds. Still other examples of conditions for respiration sensor activation include the presence of specified electrocardiogram characteristics, the status of therapy delivery (e.g. anti-tachyarrhythmia pacing), a detected drop in intracardiac impedance that is below a specified threshold value, or manual activation by a user or healthcare provider.

At **504,** the RAC measure is determined during the tachyarrhythmia. In an example, determination of the RAC measure can occur on an ongoing basis. In an example, determination of the RAC measure can occur only if specified respiration sensor activation conditions are met, such as those described above with respect to FIG. **4****.** In an example, the RAC measure can be determined after a specified time period, such as 5-10 seconds after the onset of the tachyarrhythmia. In an example, the RAC measure can be determined over multiple cardiac cycles. At **506,** if the respiration characteristic is not increased above a third threshold value, or if the RAC measurement is not decreased such that it is below a fourth threshold value, then at **422** the tachyarrhythmia is declared hemodynamically stable. At **506,** if the respiration characteristic is increased above a third threshold value, and if the RAC measurement is decreased such that it is below a fourth threshold value, then at **426** the tachyarrhythmia is declared hemodynamically unstable. In an example, the fourth threshold value can be a specified percentage of the baseline RAC measure, such as 80%. After the tachyarrhythmia has been declared hemodynamically stable at **422** or unstable at **426,** the process flows back to **412** and **414.** At **412,** if the tachyarrhythmia has been declared hemodynamically stable, then at **428** anti-tachyarrhythmia pacing (ATP) is provided to the patient. At **412,** if the tachyarrhythmia has been declared hemodynamically unstable, then at **430** shock therapy is provided to the patient. At **414,** if the tachyarrhythmia has been declared hemodynamically stable, then at **432** ATP is provided to the patient. At **414,** if the tachyarrhythmia has been declared hemodynamically unstable, then at **434** cardioversion is provided to the patient.

### Additional Notes

The above detailed description includes references to the accompanying drawings, which form a part of the detailed description.

The above description is intended to be illustrative, and not restrictive. For example, the above-described examples (or one or more aspects thereof) may be used in combination with each other. Other embodiments can be used, such as by one of ordinary skill in the art upon reviewing the above description. The scope of the invention should be determined with reference to the appended claims.

## Claims

1. An apparatus comprising:
a cardiac rhythm management device (100) comprising:
a tachyarrhythmia detection circuit (204) configured to detect whether tachyarrhythmia is present in a subject;
a respiration sensing circuit, coupled to the tachyarrhythmia detection circuit, configured to sense a respiration signal from the subject;
an auxiliary sensing circuit, coupled to the tachyarrhythmia detection circuit, configured to sense an auxiliary physiological signal from the subject, the auxiliary physiological signal being indicative of a physical activity level; and
a processor circuit (212), coupled to the respiration sensing circuit and the auxiliary sensing circuit, the processor circuit configured to:
determine a characteristic of the respiration signal;
determine a characteristic of the auxiliary physiological signal;
determine a relationship between the characteristic of the respiration signal and the characteristic of the auxiliary physiological signal; and
use the relationship to determine a hemodynamic stability characteristic of the tachyarrhythmia,
**characterised in that** the processor is further configured to:
compare the characteristic of the respiration signal to a first threshold value;
compare the characteristic of the auxiliary physiological signal to a second threshold value; and
use the comparisons to determine an indication of at least one of concordance or discordance,
wherein the processor is configured to determine discordance when the characteristic of the respiration signal exceeds the first threshold value and the characteristic of the auxiliary physiological signal is less than the second threshold value,
wherein the processor is configured to declare that the tachyarrhythmia is hemodynamically unstable in response to a determined discordance,
wherein the processor is configured to determine concordance when: 1) the characteristic of the respiration signal exceeds the first threshold value and the characteristic of the auxiliary physiological signal exceeds the second threshold value; or 2) the characteristic of the respiration signal is less than the first threshold value and the characteristic of the auxiliary physiological signal is less than the second threshold value,
wherein the processor is configured to declare that the tachyarrhythmia is hemodynamically stable in response to a determined concordance.

2. The apparatus of any one of the preceding claims, wherein the processor circuit is configured to determine the characteristic of the respiration signal and the characteristic of the auxiliary physiological signal, respectively, determined over multiple cardiac cycles.

3. The apparatus of any one of the preceding claims, wherein the auxiliary sensing circuit is configured to sense at least one of a heart rate, a heart sound, or an acceleration.

4. The apparatus of any one of the preceding claims, wherein the processor is configured to determine the characteristic of the respiration signal by determining at least one of: a time rate of change of a respiration rate, a time rate of change of a respiration depth, or a time rate of change of a respiration morphological pattern;
and wherein the processor is configured to determine the characteristic of the respiration signal using the characteristic of the respiration signal determined over multiple cardiac cycles.

5. The apparatus of any one of the preceding claims, wherein the processor is configured to communicate an indication of the hemodynamic stability characteristic of the tachyarrhythmia to a user interface (121, 124) or process.

6. The apparatus of any one of the preceding claims, wherein the processor is configured to use the hemodynamic stability characteristic of the tachyarrhythmia to control therapy provided to the subject.

7. The apparatus of claim 6, comprising a therapy circuit, coupled to the processor circuit, the therapy circuit configured to provide anti-tachyarrhythmia pacing to the subject when the tachyarrhythmia is classified as ventricular tachyarrhythmia and is hemodynamically stable.

8. The apparatus of claim 6, comprising a therapy circuit, coupled to the processor circuit, the therapy circuit configured to provide shock therapy to the subject when the tachyarrhythmia is classified as ventricular tachyarrhythmia and is hemodynamically unstable.

9. The apparatus of claim 8, wherein the therapy circuit is configured to withhold shock therapy when the tachyarrhythmia is classified as ventricular tachyarrhythmia and is hemodynamically stable.

## Patentansprüche

1. Vorrichtung, welche aufweist:
eine Herzrhythmus-Managementvorrichtung (100), welche aufweist:
eine Tachyarrhythmie-Erfassungsschaltung (204), die konfiguriert ist zum Erfassen, ob eine Tachyarrhythmie in einem Subjekt auftritt;
eine Atmungserfassungsschaltung, die mit der Tachyarrhythmie-Erfassungsschaltung gekoppelt und zum Erfassen eines Atmungssignals von dem Subjekt konfiguriert ist;
eine Hilfserfassungsschaltung, die mit der Tachyarrhythmie-Erfassungsschaltung gekoppelt und zum Erfassen eines physiologischen Hilfssignals von dem Subjekt konfiguriert ist, wobei das physiologische Hilfssignal einen physischen Aktivitätspegel anzeigt; und
eine Prozessorschaltung (212), die mit der Atmungserfassungsschaltung und der Hilfserfassungsschaltung gekoppelt ist, welche Prozessorschaltung konfiguriert ist zum:
Bestimmen einer Charakteristik des Atmungssignals;
Bestimmen einer Charakteristik des physiologischen Hilfssignals;
Bestimmen einer Beziehung zwischen der Charakteristik des Atmungssignals und der Charakteristik des physiologischen Hilfssignals; und
Verwenden der Beziehung zum Bestimmen einer hämodynamischen Stabilitätscharakteristik der Tachyarrhythmie,
**dadurch gekennzeichnet, dass**
der Prozessor weiterhin konfiguriert ist zum Vergleichen der Charakteristik des Atmungssignals mit einem ersten Schwellenwert;
Vergleichen der Charakteristik des physiologischen Hilfssignals mit einem zweiten Schwellenwert; und
Verwenden der Vergleichsergebnisse zum Bestimmen einer Anzeige von zumindest einer von Übereinstimmung oder Nichtübereinstimmung,
wobei der Prozessor konfiguriert ist zum Bestimmen von Nichtübereinstimmung, wenn die Charakteristik des Atmungssignals den ersten Schwellenwert überschreitet und die Charakteristik des physiologischen Hilfssignals kleiner als der zweite Schwellenwert ist,
wobei der Prozessor konfiguriert ist zum Erklären, dass die Tachyarrhythmie hämodynamisch instabil ist, als Antwort auf eine bestimmte Nichtübereinstimmung,
bei der der Prozessor konfiguriert ist zum Bestimmen einer Übereinstimmung, wenn: 1) die Charakteristik des Atmungssignals den ersten Schwellenwert überschreitet und die Charakteristik des physiologischen Hilfssignals den zweiten Schwellenwert überschreitet; oder 2) die Charakteristik des Atmungssignals kleiner als der erste Schwellenwert ist und die Charakteristik des physiologischen Hilfssignals kleiner als der zweite Schwellenwert ist,
wobei der Prozessor konfiguriert ist zum Erklären, dass die Tachyarrhythmie hämodynamisch stabil ist, als Antwort auf eine bestimmte Übereinstimmung.

2. Vorrichtung nach einem der vorhergehenden Ansprüche, bei der die Prozessorschaltung konfiguriert ist zum Bestimmen der Charakteristik des Atmungssignals bzw. der Charakteristik des physiologischen Hilfssignals, die jeweils über mehrere Herzzyklen bestimmt sind.

3. Vorrichtung nach einem der vorhergehenden Ansprüche, bei dem die Hilfserfassungsschaltung konfiguriert ist zum Erfassen von zumindest einer/einem von einer Herzfrequenz, einem Herzton oder einer Beschleunigung.

4. Vorrichtung nach einem der vorhergehenden Ansprüche, bei der der Prozessor konfiguriert ist zum Bestimmen der Charakteristik des Atmungssignals durch Bestimmen von zumindest einer von: einer zeitlichen Rate der Änderung einer Atmungsfrequenz, einer zeitlichen Rate der Änderung einer Atmungstiefe oder einer zeitlichen Rate der Änderung eines morphologischen Atmungsmusters;
wobei der Prozessor konfiguriert ist zum Bestimmen der Charakteristik des Atmungssignals unter Verwendung der Charakteristik des über mehrere Herzzyklen bestimmten Atmungssignals.

5. Vorrichtung nach einem der vorhergehenden Ansprüche, bei der der Prozessor konfiguriert ist zum Kommunizieren einer Anzeige der hämodynamischen Stabilitätscharakteristik der Tachyarrhythmie zu einer Benutzerschnittstelle (121, 124) oder einem Prozess.

6. Vorrichtung nach einem der vorhergehenden Ansprüche, bei der der Prozessor konfiguriert ist zum Verwenden der hämodynamischen Stabilitätscharakteristik der Tachyarrhythmie zur Steuerung einer dem Subjekt verabreichten Therapie.

7. Vorrichtung nach Anspruch 6, aufweisend eine Therapieschaltung, die mit der Prozessorschaltung gekoppelt ist, wobei die Therapieschaltung konfiguriert ist, dem Subjekt eine Antitachyarrhythmie-Schrittgabe zuzuführen, wenn die Tachyarrhythmie als eine ventrikuläre Tachyarrhythmie klassifiziert und hämodynamisch stabil ist.

8. Vorrichtung nach Anspruch 6, aufweisend eine Therapieschaltung, die mit der Prozessorschaltung gekoppelt ist, wobei die Therapieschaltung konfiguriert ist, dem Subjekt eine Schocktherapie zuzuführen, wenn die Tachyarrhythmie als ventrikuläre Tachyarrhythmie klassifiziert und hämodynamisch instabil ist.

9. Vorrichtung nach Anspruch 8, bei der die Therapieschaltung konfiguriert ist, eine Schocktherapie zurückzuhalten, wenn die Tachyarrhythmie als ventrikuläre Tachyarrhythmie klassifiziert und hämodynamisch stabil ist.

## Revendications

1. Appareil comprenant :
un dispositif de gestion de rythme cardiaque (100) qui comprend :
un circuit de détection de tachyarythmie (204) qui est configuré de manière à ce qu'il détecte si oui ou non une tachyarythmie est présente chez un sujet ;
un circuit de détection de respiration, qui est couplé au circuit de détection de tachyarythmie, qui est configuré de manière à ce qu'il détecte un signal de respiration en provenance du sujet ;
un circuit de détection auxiliaire, qui est couplé au circuit de détection de tachyarythmie, qui est configuré de manière à ce qu'il détecte un signal physiologique auxiliaire en provenance du sujet, le signal physiologique auxiliaire étant indicatif d'un niveau d'activité physique ; et
un circuit de processeur (212), qui est couplé au circuit de détection de respiration et au circuit de détection auxiliaire, le circuit de processeur étant configuré de manière à ce qu'il réalise les opérations qui suivent :
la détermination d'une caractéristique du signal de respiration ;
la détermination d'une caractéristique du signal physiologique auxiliaire ;
la détermination d'une relation entre la caractéristique du signal de respiration et la caractéristique du signal physiologique auxiliaire ; et
l'utilisation de la relation de manière à déterminer une caractéristique de stabilité hémodynamique de la tachyarythmie ;
**caractérisé en ce que** le processeur est en outre configuré de manière à ce qu'il réalise les opérations qui suivent :
la comparaison de la caractéristique du signal de respiration avec une première valeur de seuil ;
la comparaison de la caractéristique du signal physiologique auxiliaire avec une seconde valeur de seuil ; et
l'utilisation des comparaisons de manière à déterminer une indication d'au moins soit une concordance, soit une discordance ; dans lequel :
le processeur est configuré de manière à ce qu'il détermine une discordance lorsque la caractéristique du signal de respiration excède la première valeur de seuil et que la caractéristique du signal physiologique auxiliaire est inférieure à la seconde valeur de seuil ; dans lequel :
le processeur est configuré de manière à ce qu'il déclare que la tachyarythmie est instable hémodynamiquement en réponse à la détermination d'une discordance ; dans lequel :
le processeur est configuré de manière à ce qu'il détermine une concordance lorsque : 1) la caractéristique du signal de respiration excède la première valeur de seuil et la caractéristique du signal physiologique auxiliaire excède la seconde valeur de seuil ; ou 2) la caractéristique du signal de respiration est inférieure à la première valeur de seuil et la caractéristique du signal physiologique auxiliaire est inférieure à la seconde valeur de seuil ; et dans lequel :
le processeur est configuré de manière à ce qu'il déclare que la tachyarythmie est stable hémodynamiquement en réponse à la détermination d'une concordance.

2. Appareil selon l'une quelconque des revendications qui précèdent, dans lequel le circuit de processeur est configuré de manière à ce qu'il détermine la caractéristique du signal de respiration et la caractéristique du signal physiologique auxiliaire, de façon respective, la détermination étant effectuée sur de multiples cycles cardiaques.

3. Appareil selon l'une quelconque des revendications qui précèdent, dans lequel le circuit de détection auxiliaire est configuré de manière à ce qu'il détecte au moins un paramètre pris parmi une fréquence cardiaque, un bruit du coeur et une accélération.

4. Appareil selon l'une quelconque des revendications qui précèdent, dans lequel :
le processeur est configuré de manière à ce qu'il détermine la caractéristique du signal de respiration en déterminant au moins un paramètre pris parmi : un cadencement de variation d'une fréquence de la respiration, un cadencement de variation d'une profondeur ou amplitude de la respiration, et un cadencement de variation d'un motif morphologique de la respiration ; et dans lequel :
le processeur est configuré de manière à ce qu'il détermine la caractéristique du signal de respiration en utilisant la caractéristique du signal de respiration qui est déterminée sur de multiples cycles cardiaques.

5. Appareil selon l'une quelconque des revendications qui précèdent, dans lequel le processeur est configuré de manière à ce qu'il communique une indication de la caractéristique de stabilité hémodynamique de la tachyarythmie à une interface utilisateur (121, 124) ou à un procédé.

6. Appareil selon l'une quelconque des revendications qui précèdent, dans lequel le processeur est configuré de manière à ce qu'il utilise la caractéristique de stabilité hémodynamique de la tachyarythmie pour commander/contrôler une thérapie qui est administrée au sujet.

7. Appareil selon la revendication 6, comprenant un circuit de thérapie, qui est couplé au circuit de processeur, le circuit de thérapie étant configuré de manière à ce qu'il administre une stimulation cardiaque anti-tachyarythmie au sujet lorsque la tachyarythmie est classifiée en tant que tachyarythmie ventriculaire et qu'elle est hémodynamiquement stable.

8. Appareil selon la revendication 6, comprenant un circuit de thérapie, qui est couplé au circuit de processeur, le circuit de thérapie étant configuré de manière à ce qu'il administre une thérapie par (électro)choc(s) au sujet lorsque la tachyarythmie est classifiée en tant que tachyarythmie ventriculaire et qu'elle est hémodynamiquement instable.

9. Appareil selon la revendication 8, dans lequel le circuit de thérapie est configuré de manière à ce qu'il suspende la thérapie par (électro)choc(s) lorsque la tachyarythmie est classifiée en tant que tachyarythmie ventriculaire et qu'elle est hémodynamiquement stable.
